# EUROPEAN PATENT APPLICATION

(11) **EP 4 063 500 A1**
(43) Date of publication of application: **28.09.2022**
(21) Application number: 20890516.6
(22) Date of filing: 18.11.2020
(51) Int. Cl.: C12N 9/22, C07K 14/00, C07K 19/00, C12N 15/09, C12N 5/10, C12N 15/90

(54) **GENE EDITING SYSTEM DERIVED FROM FLAVOBACTERIA**

(30) Priority: 18.11.2019 CN 201911126348
(71) Applicant: Shanghai Bluecross Medical Science Institute, Shanghai 200120 (CN)
(72) Inventor: GAO, Caixia, Beijing 100101 (CN); JIN, Shuai, Beijing 100101 (CN)
(74) Representative: Michalski Hüttermann & Partner Patentanwälte mbB
(86) International application number: PCT/CN2020/129665
(87) International publication number: WO 2021/098709

(57) **Abstract**

The present invention belongs to the field of genetic engineering. Specifically, the present invention relates to a gene editing system derived from Flavobacterium and uses thereof.

## Description

### Technical field

The present invention belongs to the field of genetic engineering. Specifically, the present invention relates to a gene editing system derived from Flavobacterium and uses thereof.

### Background

Genome editing technology is a genetic engineering technology based on the targeted modification of the genome by artificial nucleases, and is playing an increasingly powerful role in agricultural and medical research. Clustered regularly interspaced short palindromic repeats/CRISPR associated (CRISPR) systems are currently the most widely used genome editing tools. Proteins can target any location in the genome, resulting in a double-strand break (DSB) at the targeted sequence, activating intracellular Non-homologous End Joining (NHEJ) or homologous repair (Homology Directly Repair, HDR) pathways to introduce mutations. The most commonly used Cas protein is the Cas9 protein derived from *Streptococcus pyogenes*, which belongs to the Type II-A subtype in the Class II CRISPR system. Cong et al. (Multiplex Genome Engineering Using CRISPR/Cas Systems, Science, 2013) and Mali et al. (RNA-guided human genome engineering via Cas9, Science, 2013) successfully applied the CRISPR/Cas9 system in human cell lines.

Both the CRISPR/Cas12a system and the CRISPR/Cas9 system belong to the Class II CRISPR system. Zetsche et al. applied the Cas12a protein (formerly Cpf1) derived from *Streptococcus aminoacids* and *Lachnospira* for the first time to gene editing of animal cells (Cpf1 is a Single RNA -Guided Endonuclease of a Class 2 CRISPR-Cas System, Cell, 2015). The difference is that the CRISPR/Cas12a system belongs to the Type V type, which has a shorter crRNA sequence and higher specificity. The advantages further expand the gene editing toolbox of the CRISPR system.

So far, gene editing tools based on CRISPR/Cas9 and CRISPR/Cas12 have been successfully used in animal cell lines, animal individuals, plant cells, plant individuals and microorganisms. The tools have caused a revolution in the field of gene editing. However, the working efficiency of the CRISPR/Cas12a system varies greatly at different target sites, and the working efficiency is lower at some sites in the plant genome, which may be due to the fact that the existing Cas12a systems are mainly derived from pathogenic bacteria of human or animal and the optimal working temperature is higher than that in plants. Therefore, it is necessary to identify and develop a CRISPR/Cas12a system that can work stably at suitable plant temperatures.

### Brief description of the invention

The inventors found the unreported FbCas12a protein in plant symbiotic bacteria through homologous similarity comparison, and artificially predicted the mature form of its own crRNA, and compared its own crRNA with the crRNA of LbCas12a in vivo efficiency. It was found that FbCas12a can work in plant cells and has higher editing efficiency when using the crRNA of LbCas12a.

### Brief Description of Drawings

Figure 1. Alignment results of DR sequences and protein sequences of Cas12a from different sources.
Figure 2. Schematic diagram of the carrier used in the examples.
Figure 3. Editing of the rice endogenous gene OsEPSPS by the combination of FbCas12a and FbcrRNA.
Figure 4. The result of the combination of FbCas12a and FbcrRNA or LbcrRNA on rice endogenous gene editing.

### Detailed description of the invention

### 1. Definition

In the present invention, unless indicated otherwise, the scientific and technological terminologies used herein refer to meanings commonly understood by a person skilled in the art. Also, the terminologies and experimental procedures used herein relating to protein and nucleotide chemistry, molecular biology, cell and tissue cultivation, microbiology, immunology, all belong to terminologies and conventional methods generally used in the art. For example, the standard DNA recombination and molecular cloning technology used herein are well known to a person skilled in the art, and are described in details in the following references: Sambrook, J., Fritsch, E.F.and Maniatis, T., Molecular Cloning: A Laboratory Manual; Cold Spring Harbor Laboratory Press: Cold Spring Harbor, 1989. In the meantime, in order to better understand the present invention, definitions and explanations for the relevant terminologies are provided below.

As used herein, the term "and/or" encompasses all combinations of items connected by the term, and each combination should be regarded as individually listed herein. For example, "A and/or B" covers "A", "A and B", and "B". For example, "A, B, and/or C" covers "A", "B", "C", "A and B", "A and C", "B and C", and "A and B and C".

When the term "comprise" is used herein to describe the sequence of a protein or nucleic acid, the protein or nucleic acid may consist of the sequence, or may have additional amino acids or nucleotide at one or both ends of the protein or nucleic acid, but still have the activity described in this invention. In addition, those skilled in the art know that the methionine encoded by the start codon at the N-terminus of the polypeptide will be retained under certain practical conditions (for example, when expressed in a specific expression system), but does not substantially affect the function of the polypeptide. Therefore, when describing the amino acid sequence of specific polypeptide in the specification and claims of the present application, although it may not include the methionine encoded by the start codon at the N-terminus, the sequence containing the methionine is also encompassed, correspondingly, its coding nucleotide sequence may also contain a start codon; vice versa.

"Genome" as used herein encompasses not only chromosomal DNA present in the nucleus, but also organelle DNA present in the subcellular components (e.g., mitochondria, plastids) of the cell.

As used herein, "organism" includes any organism that is suitable for genome editing, eukaryotes are preferred. Examples of organisms include, but are not limited to, mammals such as humans, mice, rats, monkeys, dogs, pigs, sheep, cattle, cats; poultry such as chickens, ducks, geese; plants including monocots and dicots such as rice, corn, wheat, sorghum, barley, soybean, peanut, Arabidopsis and the like.

A "genetically modified organism" or "genetically modified cell" includes the organism or the cell which comprises within its genome an exogenous polynucleotide or a modified gene or expression regulatory sequence. For example, the exogenous polynucleotide is stably integrated within the genome of the organism or the cell such that the polynucleotide is passed on to successive generations. The exogenous polynucleotide may be integrated into the genome alone or as part of a recombinant DNA construct. The modified gene or expression regulatory sequence means that, in the organism genome or the cell genome, said sequence comprises one or more nucleotide substitution, deletion, or addition.

The term "exogenous" with respect to sequence means a sequence that originates from a foreign species, or, if from the same species, is substantially modified from its native form in composition and/or genomic locus by deliberate human intervention.

"Polynucleotide", "nucleic acid sequence", "nucleotide sequence", or "nucleic acid fragment" are used interchangeably to refer to a polymer of RNA or DNA that is single- or double-stranded, optionally containing synthetic, non-natural or altered nucleotide bases. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

"Polypeptide", "peptide", "amino acid sequence" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The terms "polypeptide", "peptide", "amino acid sequence", and "protein" are also inclusive of modifications including, but not limited to, glycosylation, lipid attachment, sulfation, gamma-carboxylation of glutamic acid residues, hydroxylation and ADP-ribosylation.

Sequence "identity" has recognized meaning in the art, and the percentage of sequence identity between two nucleic acids or polypeptide molecules or regions can be calculated using the disclosed techniques. Sequence identity can be measured along the entire length of a polynucleotide or polypeptide or along a region of the molecule. (See, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many methods for measuring the identity between two polynucleotides or polypeptides, the term "identity" is well known to the skilled person (Carrillo, H. & Lipman, D., SIAM J Applied Math 48: 1073 (1988)).

Suitable conserved amino acid replacements in peptides or proteins are known to those skilled in the art and can generally be carried out without altering the biological activity of the resulting molecule. In general, one skilled in the art recognizes that a single amino acid replacement in a non-essential region of a polypeptide does not substantially alter biological activity (See, for example, Watson et al., Molecular Biology of the Gene, 4th Edition, 1987, The Benjamin/Cummings Pub. co., p.224).

As used herein, an "expression construct" refers to a vector suitable for expression of a nucleotide sequence of interest in an organism, such as a recombinant vector. "Expression" refers to the production of a functional product. For example, the expression of a nucleotide sequence may refer to transcription of the nucleotide sequence (such as transcribe to produce an mRNA or a functional RNA) and/or translation of RNA into a protein precursor or a mature protein.

"Expression construct" of the invention may be a linear nucleic acid fragment, a circular plasmid, a viral vector, or, in some embodiments, an RNA that can be translated (such as an mRNA).

"Expression construct" of the invention may comprise regulatory sequences and nucleotide sequences of interest that are derived from different sources, or regulatory sequences and nucleotide sequences of interest derived from the same source, but arranged in a manner different than that normally found in nature.

"Regulatory sequence" or "regulatory element" are used interchangeably and refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or a tissue-specific promoter or a developmentally-regulated promoter or an inducible promoter.

"Constitutive promoter" refers to a promoter that may cause expression of a gene in most circumstances in most cell types. "Tissue-specific promoter" and "tissue-preferred promoter" are used interchangeably, and refer to a promoter that is expressed predominantly but not necessarily exclusively in one tissue or organ, but that may also be expressed in one specific cell or cell type. "Developmentally regulated promoter" refers to a promoter whose activity is determined by developmental events. "Inducible promoter" selectively expresses a DNA sequence operably linked to it in response to an endogenous or exogenous stimulus (environment, hormones, or chemical signals, and so on).

As used herein, the term "operably linked" means that a regulatory element (for example but not limited to, a promoter sequence, a transcription termination sequence, and so on) is associated to a nucleic acid sequence (such as a coding sequence or an open reading frame), such that the transcription of the nucleotide sequence is controlled and regulated by the transcriptional regulatory element. Techniques for operably linking a regulatory element region to a nucleic acid molecule are known in the art.

"Introduction" of a nucleic acid molecule (e.g., plasmid, linear nucleic acid fragment, RNA, etc.) or protein into an organism means that the nucleic acid or protein is used to transform a cell of the organism such that the nucleic acid or protein functions in the cell. As used in the present invention, "transformation" includes both stable and transient transformations.

"Stable transformation" refers to the introduction of an exogenous nucleotide sequence into the genome, resulting in the stable inheritance of foreign genes. Once stably transformed, the exogenous nucleic acid sequence is stably integrated into the genome of the organism and any of its successive generations.

"Transient transformation" refers to the introduction of a nucleic acid molecule or protein into a cell, performing its function without the stable inheritance of an exogenous gene. In transient transformation, the exogenous nucleic acid sequence is not integrated into the genome.

"Trait" refers to the physiological, morphological, biochemical, or physical characteristics of a cell or an organism.

"Agronomic trait" is a measurable parameter including but not limited to, leaf greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen uptake, root lodging, harvest index, stalk lodging, plant height, ear height, ear length, disease resistance, cold resistance, salt tolerance, and tiller number and so on.

### 2. Genome editing system based on Flavobacterium Cas12a protein

In one aspect, the present invention provides a novel Cas12a protein which comprises
(i) an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or even 100% sequence identity to SEQ ID NO: 1, or
(ii) an amino acid sequence having one or more, such as 1, 2, 3, 4, 5, 6, 7, 8, 9 or 10 amino acid substitutions, deletions or additions as compared to SEQ ID NO: 1.

"Cas12a protein", "Cas12a nuclease" and "Cas12a" are used interchangeably herein to refer to an RNA-guided nuclease or variant thereof including the Cas12a protein or a fragment thereof. Cas12a is a component of the CRISPR-Cas12a genome editing system, which can target and/or cleave a DNA target sequence under the guidance of guide RNA (crRNA) to form a DNA double-strand break (DSB). The Cas12a protein of the present invention is derived from plant symbiotic bacteria and, therefore, is particularly suitable for genome editing in plants.

In some embodiments of various aspects herein, the Cas12a protein is derived from a species from *Flavobacterium.* In some embodiments, the Cas12a protein is derived from *Flavobacterium branchiophilum.* Those skilled in the art will understand that Cas12a proteins in different strains of a same bacterial species may have certain differences in amino acid sequence, but can achieve substantially the same function.

In some embodiments of various aspects of the invention, the Cas12a protein is recombinantly produced. In some embodiments of various aspects of the invention, the Cas12a protein further contains a fusion tag, e.g., a tag used for Cas12a protein isolation/or purification. Methods for recombinant production of proteins are known in the art. A variety of tags that can be used to isolate and/or purify proteins are known in the art, including but not limited to His tags, GST tags, and the like. Generally, these tags do not alter the activity of the protein of interest. In some embodiments, the Cas12a protein is also fused with other functional proteins, such as deaminase, transcriptional activator/repressor protein, etc., so as to realize base editing or transcriptional regulation functions.

In some embodiments of various aspects of the invention, the Cas12a proteins of the invention further comprise a nuclear localization sequence (NLS), e.g., is linked to a nuclear localization sequence through a linker. The a "linker" may be a non-functional amino acid sequence which is 1-50 (e.g., 1, 2, 3, 4, 5, 6, 7, 8, 9, 10, 11, 12, 13, 14, 15, 16, 17, 18, 19, 20 or 20-25 or 25-50) or more amino acids in length and is free of secondary or higher structures. For example, the linker can be a flexible linker such as SGGS (SEQ ID NO:3). In general, one or more NLSs in the Cas12a protein should be of sufficient strength to drive the accumulation of the Cas12a protein in the nucleus in an amount that enables its genome editing function. In general, the strength of nuclear localization activity is determined by the number, location of NLSs in the Cas12a protein, one or more specific NLSs used, or a combination of these factors. Exemplary nuclear localization sequences include, but are not limited to, the SV40 nuclear localization signal sequence (e.g., set forth in SEQ ID NO:4), the nucleoplasmin nuclear localization signal sequence (e.g., set forth in SEQ ID NO:5). In addition, according to the DNA position to be edited, the Cas12a protein of the present invention may also include other localization sequences, such as cytoplasmic localization sequences, chloroplast localization sequences, mitochondrial localization sequences, and the like. In some embodiments, the plurality of localization sequences can be linked by linkers. In some specific embodiments, the Cas12a protein comprises the amino acid sequence shown in SEQ ID NO:6.

In one aspect, the present invention provides the use of the Cas12a protein of the present invention for genome editing in a cell, preferably a eukaryotic cell, more preferably a plant cell.

In one aspect, the present invention provides a genome editing system for site-directed modification of a target nucleic acid sequence in the genome of a cell, comprising the Cas12a protein of the present invention and/or an expression construct comprising a nucleotide sequence encoding the Cas12a protein of the present invention.

As used herein, the "genome editing system" and "gene editing system" are interchangeable used and refers to a combination of components required for genome editing of the genome in a cell. The individual components, e.g., the Cas12aprotein, the gRNA, or the corresponding expression construct, etc., of the system may be present independently of each other, or may be present in any combination as a composition.

In some embodiments, the genome editing system further comprises at least one guide RNA (gRNA) and/or an expression construct comprising a nucleotide sequence encoding the at least one guide RNA.

"Guide RNA" and "gRNA" are used interchangeably herein. The guide RNA of the CRISPR-Cas12a genome editing system is usually composed of only a crRNA molecule, wherein the crRNA contains sufficient identity to the target sequence to hybridize with the complement sequence of the target sequence and guides the CRISPR complex (Cas12a+crRNA) to bind with the target sequence in a sequence specific manner.

In some embodiments of the methods of the invention, the guide RNA is a crRNA. In some embodiments, the guide RNA comprises the crRNA scaffold sequence set forth in SEQ ID NO: 10 or 11. In some preferred embodiments, the crRNA scaffold sequence is SEQ ID NO:11. In some embodiments, the cRNA sequence further includes a sequence (i.e., a spacer sequence) located at 3' of the cRNA scaffold sequence that specifically hybridizes to the complement of the target sequence.

In some embodiments, the crRNA comprises the following sequence:
i) 5'-AAUUUCUACUAUUGUAGAU (SEQ ID NO: 10)-Nx-3'; or
ii) 5'-UAAUUUCUACUAAGUGUAGAU (SEQ ID NO: 11)-Nx-3';
   wherein Nx represents a nucleotide sequence consisting of X consecutive nucleotides, and N is independently selected from A, G, C and U; X is an integer of 18≤X≤35, preferably, X=20, 21, 22 or 23. In some embodiments, the sequence Nx (spacer sequence) is capable of specifically hybridizing to the complement of the target sequence.

In general, a protospacer adjacent motif (PAM) is needed at the 5' of the target sequence to be targeted by the genome editing system of the present invention. The PAM may be, for example, 5'-TTTN, where N represents A, G, C or T. However, different PAM sequences can also be used. Based on the presence of PAMs, those skilled in the art can readily determine target sequences in the genome that can be used for targeting and optionally editing and design suitable guide RNAs accordingly. For example, if there is a PAM sequence 5'-TTTG-3' in the genome, about 18 to about 35, preferably 20, 21, 22 or 23 consecutive nucleotides immediately adjacent to its 3' can be used as the target sequence.

In some embodiments, the at least one guide RNA is encoded by different expression constructs. In some embodiments, the at least one guide RNA is encoded by a same expression construct. In some embodiments, the at least one guide RNA and the Cas12a protein of the invention are encoded by the same expression construct.

For example, in some embodiments, the genome editing system may comprise any one selected from the group consisting of:
i) the Cas12a protein of the present invention and the at least one guide RNA, optionally, the Cas12a protein and the at least one guide RNA form a complex;
ii) an expression construct comprising a nucleotide sequence encoding the Cas12a protein of the invention, and the at least one guide RNA;
iii) a Cas12a protein of the present invention, and an expression construct comprising a nucleotide sequence encoding the at least one guide RNA;
iv) an expression construct comprising a nucleotide sequence encoding the Cas12a protein of the invention, and an expression construct comprising a nucleotide sequence encoding the at least one guide RNA;
v) an expression construct comprising a nucleotide sequence encoding the Cas12a protein of the invention and a nucleotide sequence encoding the at least one guide RNA.

In order to obtain efficient expression in a cell, in some embodiments of the present invention, the nucleotide sequence encoding the Cas12a protein is codon-optimized for the organism from which the cell to be genome edited is derived.

Codon optimization refers to a method for replacing at least one codon in the natural sequence (for example, about or more than about 1, 2, 3, 4, 5, 10, 15, 20, 25, 50 or more codons) with a codon used more frequently or most frequently in the gene of the host cell, and maintaining the natural amino acid sequence while modifying the nucleic acid sequence to enhance expression in the host cell of interest. Different species exhibit specific preferences for certain codons of specific amino acids. Codon preference (difference in codon usage between organisms) is often related to the translation efficiency of messenger RNA (mRNA), which is considered as depending on the nature of the codon being translated and the availability of the specific transfer RNA (tRNA) molecule. The advantages of the selected tRNA in the cell generally reflect the codons most frequently used for peptide synthesis. Therefore, genes may be tailored to the optimal gene expression in a given organism based on codons optimization. The codon usage tables may be easily obtained, for example, in the codon usage database ("Codon Usage Database") available at www.kazusa.orjp/codon/, and these tables may be adjusted and applied in different ways. See Nakamura Y. et al., "Codon usage tabulated from the international DNA sequence databases: status for the year 2000". Nucl. Acids Res., 28: 292 (2000).

The organism from which the cell that can be genome-edited by the Cas12a protein or the genome editing system of the present invention is derived is preferably an eukaryote, including but not limited to, a mammal such as human, mouse, rat, monkey, dog, pig, sheep , cattle, cat; poultry such as chicken, duck, goose; a plant including monocotyledonous and dicotyledonous plant, such as rice, corn, wheat, sorghum, barley, soybean, peanut, Arabidopsis, etc. Preferably, the Cas12a protein or genome editing system of the present invention is particularly suitable for genome editing in plants due to being derived from plant symbiotic bacteria.

In some embodiments of the present invention, the nucleotide sequence encoding the Cas12a protein is codon-optimized for plants such as rice.

In some specific embodiments, the nucleotide sequence encoding the Cas12a protein is selected from the group consisting of SEQ ID NO:2 and SEQ ID NO:7.

In some embodiments of the invention, the nucleotide sequence encoding the Cas12a protein and/or the nucleotide sequence encoding the at least one guide RNA is operably linked to an expression regulatory element, such as a promoter.

Examples of promoters that can be used in the present invention include but are not limited to polymerase (pol) I, pol II or pol III promoters. Examples of pol I promoters include chicken RNA pol I promoter. Examples of pol II promoters include but are not limited to cytomegalovirus immediate early(CMV) promoter, rous sarcoma virus long terminal repeat(RSV-LTR) promoter and simian virus 40(SV40) immediate early promoter. Examples of pol III promoters include U6 and H1 promoter. Inducible promoter such as metalothionein promoter can be used. Other examples of promoters include T7 bacteriophage promoter, T3 bacteriophage promoter, β-galactosidase promoter and Sp6 bacteriophage promoter etc. When used for plants, promoters that can be used include but are not limited to cauliflower mosaic virus 35S promoter, maize Ubi-1 promoter, wheat U6 promoter, rice U3 promoter, maize U3 promoter and rice actin promoter etc.

In some embodiments, for precise production of guide RNA within the cell, in the expression construct comprising a nucleotide sequence encoding the at least one guide RNA, the 5' end of the guide RNA coding sequence is linked to 3' end of a first ribozyme coding sequence, wherein the first ribozyme is designed to cleave the first ribozyme-guide RNA fusion RNA transcribed in the cell at the 5' end of the guide RNA, thereby forming a guide RNA that does not carry additional nucleotide at the 5' end. In one embodiment, the 3' end of the guide RNA coding sequence is linked to the 5' end of a second ribozyme coding sequence, wherein the second ribozyme is designed to cleave the guide RNA-second ribozyme fusion RNA transcribed in the cell at the 3' end of the guide RNA, thereby forming a guide RNA that does not carry additional nucleotide at the 3' end. In some embodiments, the 5' end of the guide RNA coding sequence is linked to the 3' end of a first ribozyme coding sequence, and the 3' end of the guide RNA coding sequence is linked to the 5' end of a second ribozyme coding sequence, wherein the first ribozyme is designed to cleave the first ribozyme-guide RNA-second ribozyme fusion RNA transcribed in the cell at the 5' end of the guide RNA, and the second ribozyme is designed to cleave the first ribozyme-guide RNA-second ribozyme fusion RNA transcribed in the cell at the 3' end of the guide RNA, thereby forming a guide RNA that does not carry additional nucleotides at the 5' and 3' ends.

The design of the first or second ribozyme is within the skill of those skilled in the art. See, for example, Gao et al., JIPB, Apr, 2014; Vol 56, Issue 4, 343-349.

In one specific embodiment, the first ribozyme is encoded by the following sequence: 5'-(N)₆CTGATGAGTCCGTGAGGACGAAACGAGTAAGCTCGTC-3' (SEQ ID NO:31), wherein N is independently selected from A, G, C and T, and (N)₆ represents a sequence that is reverse complementary to the first 6 nucleotides of the 5' end of the guide RNA. In one specific embodiment, the second ribozyme is encoded by the following sequence: 5'-GGCCGGCATGGTCCCAGCCTCCTCGCTGGCGCCGGCTGGGCAACATGCTTCGGC ATGGCGAATGGGAC-3' (SEQ ID NO: 32).

In some embodiments, for precise production of guide RNA within the cell, in the expression construct comprising a nucleotide sequence encoding the at least one guide RNA, the 5' end of the guide RNA coding sequence is linked to 3' end of a first tRNA coding sequence, wherein the first tRNA is designed to cleave the first tRNA-guide RNA fusion RNA transcribed in the cell at the 5' end of the guide RNA (i.e., cleaved by the precise tRNA-processing machinery present within the cell that precisely excises the 5' and 3' extra sequences of the precursor tRNA to form the mature tRNA), thereby forming a guide RNA that does not carry additional nucleotide at the 5' end. In one embodiment, the 3' end of the guide RNA coding sequence is linked to the 5' end of a second tRNA coding sequence, wherein the second tRNA is designed to cleave the guide RNA-second tRNA fusion RNA transcribed in the cell at the 3' end of the guide RNA, thereby forming a guide RNA that does not carry additional nucleotide at the 3' end. In some embodiments, the 5' end of the guide RNA coding sequence is linked to the 3' end of a first tRNA coding sequence, and the 3' end of the guide RNA coding sequence is linked to the 5' end of a second tRNA coding sequence, wherein the first tRNA is designed to cleave the first tRNA-guide RNA-second tRNA fusion RNA transcribed in the cell at the 5' end of the guide RNA, and the second tRNA is designed to cleave the first tRNA-guide RNA-second tRNA fusion RNA transcribed in the cell at the 3' end of the guide RNA, thereby forming a guide RNA that does not carry additional nucleotides at the 5' and 3' ends.

The design of such tRNA-guide RNA fusions is within the skill of those skilled in the art. For example, see Xie et al., PNAS, Mar 17, 2015; vol. 112, no. 11, 3570-3575.

### 3. Method for site-directed modification of target nucleic acid sequence in the genome of a cell

In another aspect, the present invention provides a method for site-directed modification of a target nucleic acid sequence in the genome of a cell, comprising introducing the genome editing system of the present invention into the cell.

In some embodiments, introduction of the genome editing system results in a double-strand break (DSB) in the target nucleic acid sequence. Then, substitution, deletion and/or addition of one or more nucleotides in the target nucleic acid sequence or near the target nucleic acid sequence are achieved through the repair function of the cell.

In another aspect, the present invention also provides a method of producing a genetically modified cell, comprising introducing the genome editing system of the present invention into the cell.

In another aspect, the present invention also provides a genetically modified organism comprising the genetically modified cell produced by the method of the present invention or progeny cell thereof.

In the present invention, the target sequence to be modified may be located at any location in the genome, for example, in a functional gene such as a protein-encoding gene, or may be, for example, located in a gene expression regulatory region such as a promoter region or an enhancer region, thereby the gene functional modification or gene expression modification can be achieved. Modifications in the target sequence of the cell can be detected by T7EI, PCR/RE or sequencing methods.

In the methods of the present invention, the gene editing system can be introduced into cells by a variety of methods well known to those skilled in the art.

Methods that can be used to introduce a gene editing system of the present invention into a cell include, but are not limited to, calcium phosphate transfection, protoplast fusion, electroporation, lipofection, microinjection, viral infection (e.g., baculovirus, vaccinia virus, adenovirus, adeno-associated virus, lentivirus and other viruses), gene gun method, PEG-mediated protoplast transformation, Agrobacterium-mediated transformation.

In some embodiments, the method of the invention is performed in vitro. For example, the cell is an isolated cell, or a cell in an isolated tissue or organ.

In some other embodiments, the method of the present invention can also be performed in vivo. For example, the cell is a cell within an organism, and the system of the invention can be introduced in vivo by, for example, a virus or Agrobacterium-mediated method.

The cell that can be genome-edited by the Cas12a protein or the genome editing system of the present invention is derived from an eukaryote, including but not limited to, a mammal such as human, mouse, rat, monkey, dog, pig, sheep , cattle, cat; poultry such as chicken, duck, goose; a plant including monocotyledonous and dicotyledonous plant, such as rice, corn, wheat, sorghum, barley, soybean, peanut, Arabidopsis, etc.

Preferably, the Cas12a protein or genome editing system of the present invention is particularly suitable for genome editing in plants due to being derived from plant symbiotic bacteria.

Accordingly, the present invention provides a method of producing a genetically modified plant comprising introducing the genome editing system of the present invention into at least one plant, thereby resulting in a modification in the genome of said at least one plant. The modifications include substitution, deletion and/or addition of one or more nucleotides.

In the method of the present invention, the genome editing system may be introduced into the plant with a variety of methods well known to those skilled in the art. The methods used for introducing the genome editing system of the present invention into a plant include, but are not limited to gene gun method, PEG-mediated protoplast transformation, Agrobacterium-mediated transformation, plant virus-mediated transformation, pollen tube pathway and ovary injection method.

In the method of the present invention, the modification of the target sequence can be achieved by only introducing or producing the Cas12a protein and the guide RNA in the plant cell, and the modification can be stably inherited, without any need to stably transform the genome editing system into the plant. This avoids the potential off-target effect of the stable genome editing system and also avoids the integration of the exogenous nucleotide sequence in the plant genome, thereby providing greater biosafety.

In some preferred embodiments, the introduction is carried out in the absence of selection pressure to avoid integration of the exogenous nucleotide sequence into the plant genome.

In some embodiments, the introduction comprises transforming the genome editing system of the present invention into an isolated plant cell or tissue and then regenerating the transformed plant cell or tissue into an intact plant. Preferably, the regeneration is carried out in the absence of selection pressure, i.e., no selection agent for the selection gene on the expression vector is used during tissue culture. Avoiding the use of a selection agent can increase the regeneration efficiency of the plant, obtaining a modified plant free of exogenous nucleotide sequences.

In some other embodiments, the genome editing system of the present invention can be transformed into specific parts of an intact plant, such as leaves, shoot tips, pollen tubes, young ears or hypocotyls. This is particularly suitable for the transformation of plants that are difficult to regenerate in tissue culture.

In some embodiments of the invention, the in vitro expressed protein and/or the in vitro transcribed RNA molecule are directly transformed into the plant. The protein and/or RNA molecule is capable of performing genome editing in plant cells and is subsequently degraded by the cell, avoiding integration of the exogenous nucleotide sequence in the plant genome.

Thus, in some embodiments, genetic modification of a plant using the method of the present invention may result in a plant whose genome is free of integration of exogenous polynucleotide, i.e., a transgene-free modified plant.

In some embodiments of the present invention, the modification is associated with plant traits, such as agronomic traits. For example, the modification results in a plant having altered (preferably improved) traits, such as agronomic traits, relative to a wild type plant.

In some embodiments, the method further comprises the step of screening a plant having the desired modification and/or desired traits, such as agronomic traits.

In some embodiments of the present invention, the method further comprises the step of obtaining progenies of the genetically modified plant. Preferably, the genetically modified plant or the progenies thereof have the desired modification and/or the desired traits, such as agronomic traits.

In another aspect, the invention also provides a genetically modified plant or progenies or a part thereof, wherein the plant is obtained by the method according to the present invention as described above. In some embodiments, the genetically modified plant or the progenies or a part thereof is transgene-free. Preferably, the genetically modified plant or progenies thereof have the desired genetic modification and/or the desired traits, such as agronomic traits.

In another aspect, the present invention provides a method of plant breeding comprising crossing a first genetically modified plant obtained by the above method of the present invention with a second plant not containing the modification, thereby the genetic modification is introduced into the second plant. Preferably, the first genetically modified plant has the desired traits, such as the agronomic traits.

### 4. Kit

The present invention also includes a kit for use in the method of the present invention, the kit comprising the genome editing system of the present invention, and instructions for use. The kit generally include a label indicating the intended use and/or method for use of the contents of the kit. The term label includes any written or recorded material provided on or with the kit or otherwise provided with the kit.

### Examples

### Example 1. Discovery of CRISPR/Cas12a system from plant symbiotic bacteria using homologous similarity alignment

According to the reports of Bai et al. (Functional overlap of the Arabidopsis leaf and root microbiota) and Levy et al. (Genomic features of bacterial adaptation to plants) on plant symbiotic bacteria, the inventors collected the genome sequences of plant symbiotic bacteria and used CRISPRdisco software to analyze the CRISPR systems in 4269 plant symbiotic bacteria genomes, and CRISPR systems were found to be relatively abundant in plant symbiotic bacteria, but most of them are Type I CRISPR systems in Class I, and only one Cas12a protein is Type V in Class II. The Cas12a protein is derived from *Flavobacterium branchiophilum*, NCBI contig ID is FQ859183.1, and GeneBank protein ID is CCB70584.1, hereinafter referred to as FbCas12a.

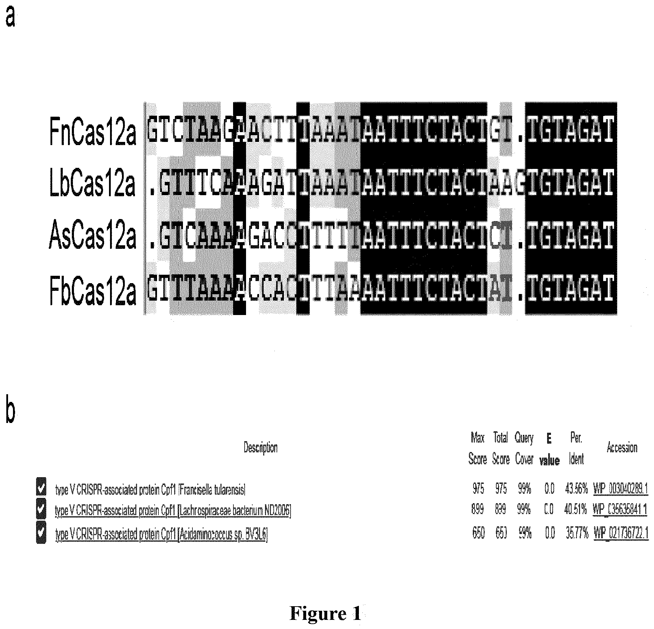

The size of the protein is 1318aa (SEQ ID NO:1), but there is no other Cas protein sequence near this sequence. The CRISPR repeat sequence begins at 1509bp downstream in the genome. There are 37 Spacer sequences in total. The Direct Repeat is GTTTAAAACCACTTTAAAATTTCTACTATTGTAGAT (SEQ ID NO: 9). The comparison with the Direct Repeat of the commonly used Cas12a proteins FnCas12a, LbCas12a, and AsCas12a is shown in Figure 1a. The protein sequence alignment result of this protein and commonly used FnCas12a, LbCas12a, and AsCas12a is shown in Figure 1b, and the sequence similarity alignment was performed using the NCBI blastp program.

### Example 2. Preparation of FbCas12a and LbCas12a expression vectors

The vectors used for plant protoplast transformation were constructed: pJIT163-UBI-FbCas12a, pJIT163-UBI-LbCas12a, pJIT163-UBI-FbcrRNA and pJIT163-UBI-LbcrRNA.

The coding sequence of FbCas12a from *Flavobacterium branchiophilum* was codon-optimized, and two nuclear localization signals (NLS) were added at the 3' end, and BamHI/SmaI restriction sites were added at the two ends, allowing better expression and localization of the FbCas12a protein in rice. The nucleotide coding sequence of FbCas12a after addition of NLSs and codon optimization is shown in SEQ ID NO: 7 in the sequence listing. In SEQ ID NO: 7, the 3967th-3987th position is the SV40 nuclear localization signal sequence, the 3988th-3999th position is the SGGS linker between the two nuclear localization signal sequences, the 4000th-4047th position is the nucleoplasmin nuclear localization signal sequence, and the 1st-3966^{th} position is the coding sequence of FbCas12a protein. SEQ ID NO:7 encodes the protein shown in SEQ ID NO:6, i.e., the FbCas12a nuclease with nuclear localization signals.

The DNA shown in SEQ ID NO: 7 with BamHI/SmaI sites was artificially synthesized. After double digestion with BamHI/SmaI, the DNA fragment was ligated into the expression vector pJIT163 (Guerineau, F., Lucy, A. & Mullineaux, P., Effect of two consensus sequences preceding the translation initiator codon on gene expression in plant protoplasts. Plant Molecular Biology 18, 815-818, 1992)(The vector is publicly available from the Institute of Genetics and Developmental Biology, Chinese Academy of Sciences), and the resulting construct was named pJIT163-FbCas12a. It was confirmed by sequencing that a nucleotide fragment with the sequence shown in SEQ ID NO: 7 was inserted between the BamH I and SmaI restriction sites of the pJIT163 expression vector.

The DNA sequence of LbCas12a commonly used in laboratory genome editing was ligated into the pJIT163 vector to obtain the pJIT163-UBI-LbCas12a vector. The construction of the vector was similar to that of pJIT163-UBI-FbCas12a. The codon-optimized nucleotide coding sequence of LbCas12a is shown in SEQ ID NO: 8 in the sequence listing.

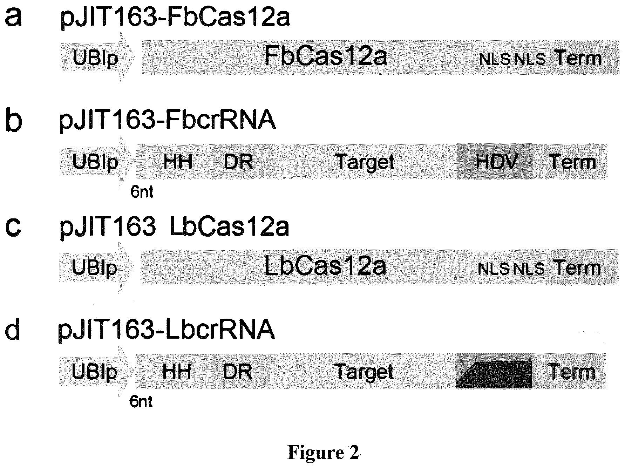

In general, pJIT163-FbCas12a and pJIT163-LbCas12a vectors contain UBI promoter, plant codon-optimized FbCas12a protein or LbCas12a coding sequence, 3' SV40 nuclear localization signal coding sequence, nucleoplasmin nuclear localization signal coding sequence, and their structures are shown in Figure 2a and Figure 2c.

### Example 3. Preparation of the vectors pJIT163-FbcrRNA with the artificially predicted mature crRNA backbone coding sequence of FbCas12a and the pJIT163-LbcrRNA

Cas12a has the crRNA self-mature function, however, unexpectedly, when the inventors used the full-length FbCas12a crRNA backbone sequence (full-length Direct Repeat), genome editing was not achieved. Therefore, it appears that FbCas12a does not mature its native crRNA backbone. The crRNA of FbCas12a needs to be explored to determine whether it can achieve genome editing.

The DNA fragment of the nucleotide sequence shown in SEQ ID NO: 14 was artificially synthesized, and the fragment contains hammerhead ribozyme (Hammerhead, HH ribozyme) and hepatitis delta virus ribozyme (Hepatitis deltavirus, HDV ribozyme), which can cleave the artificially predicted mature direct repeat (DR) corresponding to FbCas12a. Positions 1-6 of the fragment are the HindIII restriction site, positions 7-12 are the reverse complementary sequence required for HH ribozyme to work, positions 13-49 are the HH ribozyme sequence, positions 50-68 are artificially truncated DR sequence contains, and two BsaI restriction sites are contained at positions 69-88. The recognition sequence of the target sequence to be mutated in rice can be linked into the vector pJIT163-FbcrRNA through the two restriction sites. Positions 89-156 are HDV ribozyme sequence, and positions 157-162 is the SmaI restriction site sequence.

After HindIII/SmaI double digestion, the synthesized DNA fragment of SEQ ID NO: 14 was ligated into the expression vector pJIT163 to obtain the pJIT163-FbcrRNA vector. The vector contains UBI promoter, HH ribozyme, truncated FbcrRNA sequence, HDV ribozyme and CaMV terminator, and its structure is shown in Figure 2b. The pJIT163-FbcrRNA vector uses a ribozyme-based crRNA maturation strategy to obtain precisely processed crRNA sequences.

The DNA fragment of the nucleotide sequence shown in SEQ ID NO: 15 (the fragment differs from FbCas12a only in the DR sequence) was artificially synthesized, double digested by HindIII/SmaI, and ligated into the expression vector pJIT163 to obtain the pJIT163-LbcrRNA vector. A schematic diagram of the structure of the vector is shown in Figure 2d.

### Example 4. Site-directed mutation of rice endogenous gene EPSPS by FbCas12a system and mutation of four endogenous gene targets in rice using FbCas12a protein and LbCas12a crRNA

### (1) Design of the target fragments:

target-EPSPS05: TTTGGTACTAAATATACAATCCCTTGG (SEQ ID NO:16; the sequence is nucleotides 956-982 in the OsEPSPS gene of LOC_Os06g04280.1; the underlined is the PAM sequence).
target-OsCDC48: TTTATTCAGATTACATATGGTTAG (SEQ ID NO: 17; the sequence is nucleotides 582-605 in the OsCDC48 gene of LOC_Os03g05730; the underlined is the PAM sequence).
target-OsDEP1T3: TTTCAAATGGATCTAAACAGGGCCTTA (SEQ ID NO:18; the sequence is nucleotides 1919-1945 in the OsDEP1gene of LOC_Os09g26999; the underlined is the PAM sequence).
target-OsPDS: TTTGGAGTGAAATCTCTTGTCTTA(SEQ ID NO:19; the sequence is nucleotides 136-159 in the OsPDS gene of LOC_Os03g08570; the underlined is the PAM sequence).
target-OsEpspsC02: TTTATGAAAATATGTATGGAATTCATG (SEQ ID NO:20; the sequence is nucleotides 1294-1320 in the OsEPSPS gene of LOC_Os06g04280.1; the underlined is the PAM sequence).

### (2) pJIT163-FbcrRNA plasmid and pJIT163-LbcrRNA plasmid containing SP1, SP2, SP3, SP4, SP5

SP1 is the DNA encoding the RNA that can complementarily bind to the target-EPSPS05 The following single-stranded primers with sticky ends (underlined) were synthesized:
SP1-F: AGATGTACTAAATATACAATCCCTTGG (SEQ ID NO:21)
SP1-R: AAACCCAAGGGATTGTATATTTAGTAC (SEQ ID NO:22)

After primer annealing, a double-stranded DNA with sticky ends was formed, which was inserted between the two BsaI restriction sites of pJIT163-FbcrRNA to obtain a pJIT163-FbcrRNA plasmid containing SP1. The plasmid was confirmed as positive by sequencing.

SP2 to SP5 are DNAs encoding RNAs that can complementarily bind to target-OsCDC48, target-OsDEP1T3, target-OsPDS and target-OsEpspsC02.

The following single-stranded primers with sticky ends (underlined) were synthesized:
SP2-F: AGATTTCAGATTACATATGGTTAG (SEQ ID NO:23)
SP2-R: AAACCTAACCATATGTAATCTGAA (SEQ ID NO:24)
SP3-F: AGATAAATGGATCTAAACAGGGCCTTA (SEQ ID NO:25)
SP3-R: AAACATTGGCCCTGTTTAGATCCATTT (SEQ ID NO:26)
SP4-F: AGATGAGTGAAATCTCTTGTCTTA (SEQ ID NO:27)
SP4-R: AAACTAAGACAAGAGATTTCACTC (SEQ ID NO:28)
SP5-F: AGATTGAAAATATGTATGGAATTCATG (SEQ ID NO:29)
SP5-R: AAACCATGAATTCCATACATATTTTCA (SEQ ID NO:30)

After primer annealing, double-stranded DNAs with sticky ends were formed, which were inserted between the two BsaI restriction sites of pJIT163-FbcrRNA and pJIT163-LbcrRNA to obtain pJIT163-FbcrRNA plasmids and pJIT163-LbcrRNA plasmids containing SP1∼SP5. The plasmids were confirmed to be positive by sequencing.

### (3) Transformation of FbcrRNA:FbCas12a, LbcrRNA:FbCas12a, LbcrRNA:LbCas12a into rice protoplasts

The pJIT163-UBI-FbCas12a, pJIT163-UBI-FbCas12a, and pJIT163-FbcrRNA and pJIT163-LbcrRNA plasmids containing SP1~SP5 were respectively transformed into the protoplasts of rice Nipponbare. The specific process of rice protoplast transformation refers to Shan, Q. et al., Rapid and efficient gene modification in rice and Brachypodium using TALENs. Method disclosed in Molecular Plant (2013).

The genomic DNA was extracted 48 hours after rice protoplast transformation, and the DNA was used as a template to perform amplicon high-throughput sequencing to analyze the editing efficiency. The specific process of amplicon high-throughput sequencing refers to the Zhang et al. Perfectly matched 20 -nucleotide guide RNA sequences enable robust genome editing using high-fidelity SpCas9 nucleases. Methods described in Genome Biology, 2017.

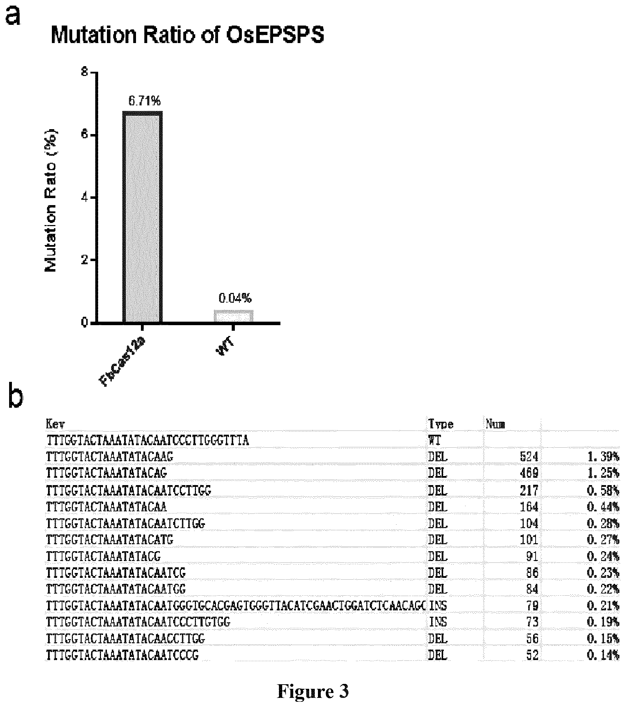

The results of the artificial matured FbcrRNA: FbCas12a high-throughput sequencing experiment are shown in Figure 3a. The results show that, compared with the control group, the FbCas12a treatment group mutated at the target site of the OsEPSPS gene, and the mutation efficiency was about 6.25%. Figure 3b shows the proportion of mutation types obtained from the analysis of high-throughput sequencing data. The results show that most of the mutation types generated by FbCas12a at the target site are deletions of DNA fragments.

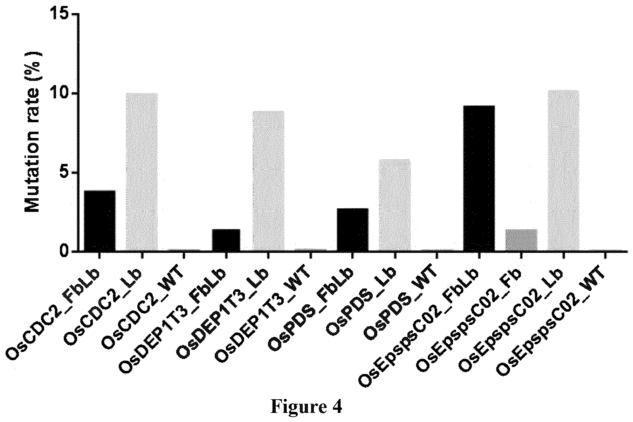

The results of another protoplast transformation experiment are shown in Figure 4. Fb represents FbcrRNA:FbCas12a, FbLb represents LbcrRNA:FbCas12a, and Lb represents LbcrRNA:LbCas12a. The results showed that FbcrRNA:FbCas12a works at all four targets in rice, but FbCas12a works more efficiently when LbcrRNA is used.

## Claims

1. A Cas12a protein which comprises
(i) an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or even 100% sequence identity to SEQ ID NO: 1, or
(ii) an amino acid sequence having substitution, deletion or addition of one or more, such as 1, 2, 3, 4, 5, 6, 7, 8, 9 or 10 amino acids as compared to SEQ ID NO: 1.

2. The Cas12a protein of claim 1, wherein the Cas12a protein is derived from a species of *Flavobacterium,* for example, the Cas12a protein is derived from *Flavobacterium branchiophilum.*

3. The Cas12a protein of claim 1 or 2, wherein the Cas12a proteins further comprise a nuclear localization sequence (NLS).

4. The Cas12a protein of claim 3, which comprises the amino acid sequence shown in SEQ ID NO:6.

5. Use of the Cas12a protein of any one of claims 1-4 for genome editing in a cell, preferably an eukaryotic cell, more preferably a plant cell.

6. A genome editing system for site-directed modification of a target nucleic acid sequence in the genome of a cell, comprising the Cas12a protein of any one of claims 1-4 and/or an expression construct comprising a nucleotide sequence encoding the Cas12a protein of any one of claims 1-4.

7. The genome editing system of claim 6, further comprises at least one guide RNA (gRNA) and/or an expression construct comprising a nucleotide sequence encoding the at least one guide RNA.

8. The genome editing system of claim 7, wherein the guide RNA is a crRNA, and comprises the crRNA scaffold sequence set forth in SEQ ID NO: 10 or 11.

9. The genome editing system of claim 7 or 8, comprises any one selected from the group consisting of:
i) the Cas12a protein of any one of claims 1-4 and the at least one guide RNA, optionally, the Cas12a protein and the at least one guide RNA form a complex;
ii) an expression construct comprising a nucleotide sequence encoding the Cas12a protein of any one of claims 1-4, and the at least one guide RNA;
iii) a Cas12a protein of any one of claims 1-4, and an expression construct comprising a nucleotide sequence encoding the at least one guide RNA;
iv) an expression construct comprising a nucleotide sequence encoding the Cas12a protein of any one of claims 1-4, and an expression construct comprising a nucleotide sequence encoding the at least one guide RNA;
v) an expression construct comprising a nucleotide sequence encoding the Cas12a protein of any one of claims 1-4 and a nucleotide sequence encoding the at least one guide RNA.

10. The genome editing system of any one of claims 6-9, wherein the nucleotide sequence encoding the Cas12a protein is codon-optimized for plants such as rice.

11. The genome editing system of claim 10, wherein the nucleotide sequence encoding the Cas12a protein is selected from the group consisting of SEQ ID NO:2 and SEQ ID NO:7.

12. The genome editing system of any one of claims 7-11, wherein the nucleotide sequence encoding the Cas12a protein and/or the nucleotide sequence encoding the at least one guide RNA is operably linked to an expression regulatory element, such as a promoter.

13. The genome editing system of any one of claims 7-11, wherein the 5' end of the guide RNA coding sequence is linked to the 3' end of a first ribozyme coding sequence, and the 3' end of the guide RNA coding sequence is linked to the 5' end of a second ribozyme coding sequence, wherein the first ribozyme is designed to cleave the first ribozyme-guide RNA-second ribozyme fusion RNA transcribed in the cell at the 5' end of the guide RNA, and the second ribozyme is designed to cleave the first ribozyme-guide RNA-second ribozyme fusion RNA transcribed in the cell at the 3' end of the guide RNA, thereby forming a guide RNA that does not carry additional nucleotides at the 5' and 3' ends.

14. The genome editing system of claim 13, wherein the first ribozyme is encoded by the sequence shown in SEQ ID NO:31, and the second ribozyme is encoded by the sequence shown in SEQ ID NO:32.

15. A method of producing a genetically modified cell, comprising introducing the genome editing system of any one of claims 6-14 into the cell.

16. The method of claim 15, wherein the cell is derived from a mammal such as human, mouse, rat, monkey, dog, pig, sheep, cattle, cat; poultry such as chicken, duck, goose; a plant including monocotyledonous and dicotyledonous plant, such as rice, corn, wheat, sorghum, barley, soybean, peanut, Arabidopsis.


Sequence listing

<110>  Institute of Genetics and Developmental Biology, Chinese Academy of
Sciences

<120>  Gene editing system derived from Flavobacterium

<130>  P2020TC1289

<150>  201911126348.4
<151>  2019-11-18

<160>  32

<170>  PatentIn version 3.5

<210>  1
<211>  1318
<212>  PRT
<213>  Flavobacterium branchiophilum

<400>  1

Met Thr Asn Lys Phe Thr Asn Gln Tyr Ser Leu Ser Lys Thr Leu Arg
1               5                   10                  15

Phe Glu Leu Ile Pro Gln Gly Lys Thr Leu Glu Phe Ile Gln Glu Lys
            20                  25                  30

Gly Leu Leu Ser Gln Asp Lys Gln Arg Ala Glu Ser Tyr Gln Glu Met
        35                  40                  45

Lys Lys Thr Ile Asp Lys Phe His Lys Tyr Phe Ile Asp Leu Ala Leu
    50                  55                  60

Ser Asn Ala Lys Leu Thr His Leu Glu Thr Tyr Leu Glu Leu Tyr Asn
65                  70                  75                  80

Lys Ser Ala Glu Thr Lys Lys Glu Gln Lys Phe Lys Asp Asp Leu Lys
                85                  90                  95

Lys Val Gln Asp Asn Leu Arg Lys Glu Ile Val Lys Ser Phe Ser Asp
            100                 105                 110

Gly Asp Ala Lys Ser Ile Phe Ala Ile Leu Asp Lys Lys Glu Leu Ile
        115                 120                 125

Thr Val Glu Leu Glu Lys Trp Phe Glu Asn Asn Glu Gln Lys Asp Ile
    130                 135                 140

Tyr Phe Asp Glu Lys Phe Lys Thr Phe Thr Thr Tyr Phe Thr Gly Phe
145                 150                 155                 160

His Gln Asn Arg Lys Asn Met Tyr Ser Val Glu Pro Asn Ser Thr Ala
                165                 170                 175

Ile Ala Tyr Arg Leu Ile His Glu Asn Leu Pro Lys Phe Leu Glu Asn
            180                 185                 190

Ala Lys Ala Phe Glu Lys Ile Lys Gln Val Glu Ser Leu Gln Val Asn
        195                 200                 205

Phe Arg Glu Leu Met Gly Glu Phe Gly Asp Glu Gly Leu Ile Phe Val
    210                 215                 220

Asn Glu Leu Glu Glu Met Phe Gln Ile Asn Tyr Tyr Asn Asp Val Leu
225                 230                 235                 240

Ser Gln Asn Gly Ile Thr Ile Tyr Asn Ser Ile Ile Ser Gly Phe Thr
                245                 250                 255

Lys Asn Asp Ile Lys Tyr Lys Gly Leu Asn Glu Tyr Ile Asn Asn Tyr
            260                 265                 270

Asn Gln Thr Lys Asp Lys Lys Asp Arg Leu Pro Lys Leu Lys Gln Leu
        275                 280                 285

Tyr Lys Gln Ile Leu Ser Asp Arg Ile Ser Leu Ser Phe Leu Pro Asp
    290                 295                 300

Ala Phe Thr Asp Gly Lys Gln Val Leu Lys Ala Ile Phe Asp Phe Tyr
305                 310                 315                 320

Lys Ile Asn Leu Leu Ser Tyr Thr Ile Glu Gly Gln Glu Glu Ser Gln
                325                 330                 335

Asn Leu Leu Leu Leu Ile Arg Gln Thr Ile Glu Asn Leu Ser Ser Phe
            340                 345                 350

Asp Thr Gln Lys Ile Tyr Leu Lys Asn Asp Thr His Leu Thr Thr Ile
        355                 360                 365

Ser Gln Gln Val Phe Gly Asp Phe Ser Val Phe Ser Thr Ala Leu Asn
    370                 375                 380

Tyr Trp Tyr Glu Thr Lys Val Asn Pro Lys Phe Glu Thr Glu Tyr Ser
385                 390                 395                 400

Lys Ala Asn Glu Lys Lys Arg Glu Ile Leu Asp Lys Ala Lys Ala Val
                405                 410                 415

Phe Thr Lys Gln Asp Tyr Phe Ser Ile Ala Phe Leu Gln Glu Val Leu
            420                 425                 430

Ser Glu Tyr Ile Leu Thr Leu Asp His Thr Ser Asp Ile Val Lys Lys
        435                 440                 445

His Ser Ser Asn Cys Ile Ala Asp Tyr Phe Lys Asn His Phe Val Ala
    450                 455                 460

Lys Lys Glu Asn Glu Thr Asp Lys Thr Phe Asp Phe Ile Ala Asn Ile
465                 470                 475                 480

Thr Ala Lys Tyr Gln Cys Ile Gln Gly Ile Leu Glu Asn Ala Asp Gln
                485                 490                 495

Tyr Glu Asp Glu Leu Lys Gln Asp Gln Lys Leu Ile Asp Asn Leu Lys
            500                 505                 510

Phe Phe Leu Asp Ala Ile Leu Glu Leu Leu His Phe Ile Lys Pro Leu
        515                 520                 525

His Leu Lys Ser Glu Ser Ile Thr Glu Lys Asp Thr Ala Phe Tyr Asp
    530                 535                 540

Val Phe Glu Asn Tyr Tyr Glu Ala Leu Ser Leu Leu Thr Pro Leu Tyr
545                 550                 555                 560

Asn Met Val Arg Asn Tyr Val Thr Gln Lys Pro Tyr Ser Thr Glu Lys
                565                 570                 575

Ile Lys Leu Asn Phe Glu Asn Ala Gln Leu Leu Asn Gly Trp Asp Ala
            580                 585                 590

Asn Lys Glu Gly Asp Tyr Leu Thr Thr Ile Leu Lys Lys Asp Gly Asn
        595                 600                 605

Tyr Phe Leu Ala Ile Met Asp Lys Lys His Asn Lys Ala Phe Gln Lys
    610                 615                 620

Phe Pro Glu Gly Lys Glu Asn Tyr Glu Lys Met Val Tyr Lys Leu Leu
625                 630                 635                 640

Pro Gly Val Asn Lys Met Leu Pro Lys Val Phe Phe Ser Asn Lys Asn
                645                 650                 655

Ile Ala Tyr Phe Asn Pro Ser Lys Glu Leu Leu Glu Asn Tyr Lys Lys
            660                 665                 670

Glu Thr His Lys Lys Gly Asp Thr Phe Asn Leu Glu His Cys His Thr
        675                 680                 685

Leu Ile Asp Phe Phe Lys Asp Ser Leu Asn Lys His Glu Asp Trp Lys
    690                 695                 700

Tyr Phe Asp Phe Gln Phe Ser Glu Thr Lys Ser Tyr Gln Asp Leu Ser
705                 710                 715                 720

Gly Phe Tyr Arg Glu Val Glu His Gln Gly Tyr Lys Ile Asn Phe Lys
                725                 730                 735

Asn Ile Asp Ser Glu Tyr Ile Asp Gly Leu Val Asn Glu Gly Lys Leu
            740                 745                 750

Phe Leu Phe Gln Ile Tyr Ser Lys Asp Phe Ser Pro Phe Ser Lys Gly
        755                 760                 765

Lys Pro Asn Met His Thr Leu Tyr Trp Lys Ala Leu Phe Glu Glu Gln
    770                 775                 780

Asn Leu Gln Asn Val Ile Tyr Lys Leu Asn Gly Gln Ala Glu Ile Phe
785                 790                 795                 800

Phe Arg Lys Ala Ser Ile Lys Pro Lys Asn Ile Ile Leu His Lys Lys
                805                 810                 815

Lys Ile Lys Ile Ala Lys Lys His Phe Ile Asp Lys Lys Thr Lys Thr
            820                 825                 830

Ser Glu Ile Val Pro Val Gln Thr Ile Lys Asn Leu Asn Met Tyr Tyr
        835                 840                 845

Gln Gly Lys Ile Ser Glu Lys Glu Leu Thr Gln Asp Asp Leu Arg Tyr
    850                 855                 860

Ile Asp Asn Phe Ser Ile Phe Asn Glu Lys Asn Lys Thr Ile Asp Ile
865                 870                 875                 880

Ile Lys Asp Lys Arg Phe Thr Val Asp Lys Phe Gln Phe His Val Pro
                885                 890                 895

Ile Thr Met Asn Phe Lys Ala Thr Gly Gly Ser Tyr Ile Asn Gln Thr
            900                 905                 910

Val Leu Glu Tyr Leu Gln Asn Asn Pro Glu Val Lys Ile Ile Gly Leu

915                 920                 925

Asp Arg Gly Glu Arg His Leu Val Tyr Leu Thr Leu Ile Asp Gln Gln
    930                 935                 940

Gly Asn Ile Leu Lys Gln Glu Ser Leu Asn Thr Ile Thr Asp Ser Lys
945                 950                 955                 960

Ile Ser Thr Pro Tyr His Lys Leu Leu Asp Asn Lys Glu Asn Glu Arg
                965                 970                 975

Asp Leu Ala Arg Lys Asn Trp Gly Thr Val Glu Asn Ile Lys Glu Leu
            980                 985                 990

Lys Glu Gly Tyr Ile Ser Gln Val  Val His Lys Ile Ala  Thr Leu Met
        995                 1000                1005

Leu Glu  Glu Asn Ala Ile Val  Val Met Glu Asp Leu  Asn Phe Gly
    1010                1015                1020

Phe Lys  Arg Gly Arg Phe Lys  Val Glu Lys Gln Ile  Tyr Gln Lys
    1025                1030                1035

Leu Glu  Lys Met Leu Ile Asp  Lys Leu Asn Tyr Leu  Val Leu Lys
    1040                1045                1050

Asp Lys  Gln Pro Gln Glu Leu  Gly Gly Leu Tyr Asn  Ala Leu Gln
    1055                1060                1065

Leu Thr  Asn Lys Phe Glu Ser  Phe Gln Lys Met Gly  Lys Gln Ser
    1070                1075                1080

Gly Phe  Leu Phe Tyr Val Pro  Ala Trp Asn Thr Ser  Lys Ile Asp
    1085                1090                1095

Pro Thr  Thr Gly Phe Val Asn  Tyr Phe Tyr Thr Lys  Tyr Glu Asn
    1100                1105                1110

Val Asp  Lys Ala Lys Ala Phe  Phe Glu Lys Phe Glu  Ala Ile Arg
    1115                1120                1125

Phe Asn  Ala Glu Lys Lys Tyr  Phe Glu Phe Glu Val  Lys Lys Tyr
    1130                1135                1140

Ser Asp  Phe Asn Pro Lys Ala  Glu Gly Thr Gln Gln  Ala Trp Thr
    1145                1150                1155

Ile Cys Thr Tyr Gly Glu Arg Ile Glu Thr Lys Arg Gln Lys Asp
    1160                1165                1170

Gln Asn Asn Lys Phe Val Ser Thr Pro Ile Asn Leu Thr Glu Lys
    1175                1180                1185

Ile Glu Asp Phe Leu Gly Lys Asn Gln Ile Val Tyr Gly Asp Gly
    1190                1195                1200

Asn Cys Ile Lys Ser Gln Ile Ala Ser Lys Asp Asp Lys Ala Phe
    1205                1210                1215

Phe Glu Thr Leu Leu Tyr Trp Phe Lys Met Thr Leu Gln Met Arg
    1220                1225                1230

Asn Ser Glu Thr Arg Thr Asp Ile Asp Tyr Leu Ile Ser Pro Val
    1235                1240                1245

Met Asn Asp Asn Gly Thr Phe Tyr Asn Ser Arg Asp Tyr Glu Lys
    1250                1255                1260

Leu Glu Asn Pro Thr Leu Pro Lys Asp Ala Asp Ala Asn Gly Ala
    1265                1270                1275

Tyr His Ile Ala Lys Lys Gly Leu Met Leu Leu Asn Lys Ile Asp
    1280                1285                1290

Gln Ala Asp Leu Thr Lys Lys Val Asp Leu Ser Ile Ser Asn Arg
    1295                1300                1305

Asp Trp Leu Gln Phe Val Gln Lys Asn Lys
    1310                1315

<210> 2
<211> 3954
<212> DNA
<213> Flavobacterium branchiophilum

<400> 2
atgaccaaca agttcacaaa tcagtactcc ctgagcaaga ccctccgctt cgagctgatt 60

ccgcagggca agacactcga gttcatccag gagaagggcc tcctgtccca ggacaagcag 120

agggccgaga gctaccagga gatgaagaag accattgaca agttccacaa gtacttcatc 180

gatctcgccc tgtcaaatgc gaagctcacc catctggaga catacctcga gctgtacaac 240

aagtctgcgg agacaaagaa ggagcagaag ttcaaggacg atctcaagaa ggtccaggac 300

aacctgagga aggagatcgt gaagtcattc tctgacggcg atgccaagag catcttcgcg 360

attctggata agaaggagct gatcaccgtg gagctggaga agtggttcga gaacaatgag 420

cagaaggaca tctacttcga tgagaagttc aagaccttca ccacatactt cacaggcttc 480

caccagaaca ggaagaatat gtactcagtc gagccaaact ctacagccat cgcgtaccgc 540

ctcattcatg agaacctccc taagttcctg gagaatgcca aggcgttcga gaagatcaag 600

caggtggagt ccctccaggt caatttccgc gagctgatgg gcgagttcgg cgacgagggc 660

ctcatcttcg tcaacgagct ggaggagatg ttccagatta actactacaa tgatgtgctc 720

agccagaacg gcatcaccat ctacaattca atcatttctg gcttcacaaa gaacgacatt 780

aagtacaagg gcctgaacga gtacatcaac aattacaatc agaccaagga caagaaggat 840

cgcctcccga agctgaagca gctctacaag cagatcctct ccgaccggat ttccctgagc 900

ttcctcccag acgccttcac cgatggcaag caggtgctga aggcgatttt cgatttctac 960

aagatcaatc tcctgtccta cacaatcgag ggccaggagg agagccagaa tctcctgctc 1020

ctgatccggc agacaattga gaacctctcc agcttcgaca cccagaagat ctacctcaag 1080

aacgatacac acctgaccac aatttcccag caggtgttcg gcgatttctc cgtcttcagc 1140

accgccctca actactggta cgagacaaaa gtgaatccga agttcgagac agagtacagc 1200

aaggcgaacg agaagaagag ggagatcctg gacaaggcca aggcggtctt caccaagcag 1260

gattacttct caattgcctt cctccaggag gtgctgtctg agtacattct caccctggac 1320

cacacatccg atatcgtcaa gaagcattca tctaactgca ttgccgacta cttcaagaat 1380

catttcgtcg cgaagaagga gaacgagaca gacaagacat tcgatttcat cgccaatatt 1440

acagcgaagt accagtgcat ccagggcatt ctcgagaacg ccgaccagta cgaggatgag 1500

ctgaagcagg accagaagct cattgataat ctgaagttct tcctcgatgc gatcctcgag 1560

ctgctgcact tcatcaagcc actccatctg aagtcagagt ctatcaccga gaaggacaca 1620

gccttctacg atgtgttcga gaactactac gaggcgctct ccctcctgac ccctctgtac 1680

aacatggtga ggaattacgt cacccagaag ccgtacagca cagagaagat caagctcaac 1740

ttcgagaatg cccagctcct gaacggctgg gacgcgaata aggagggcga ttacctcacc 1800

acaatcctga agaaggacgg caattacttc ctcgccatta tggataagaa gcacaacaag 1860

gcgttccaga agttccctga gggcaaggag aattacgaga agatggtgta caagctcctg 1920

ccaggcgtca acaagatgct ccctaaggtg ttcttctcca acaagaatat cgcctacttc 1980

aatccgagca aggagctgct cgagaactac aagaaggaga cacacaagaa gggcgacaca 2040

ttcaatctcg agcactgcca taccctgatc gacttcttca aggattccct caacaagcat 2100

gaggactgga agtacttcga tttccagttc tcagagacaa agtcttacca ggatctgagc 2160

ggcttctacc gcgaggtgga gcaccagggc tacaagatca acttcaagaa tattgactca 2220

gagtacatcg atggcctcgt caatgagggc aagctcttcc tgttccagat ctactctaag 2280

gacttctcac ctttctctaa gggcaagccg aacatgcata ccctctactg gaaggccctg 2340

ttcgaggagc agaacctcca gaatgtgatc tacaagctga atggccaggc cgagattttc 2400

ttccggaagg cgtcaatcaa gccaaagaac atcattctcc acaagaagaa gatcaagatt 2460

gcgaagaagc atttcatcga taagaagacc aagacatctg agattgtgcc tgtccagacc 2520

atcaagaacc tcaatatgta ctaccagggc aagatttccg agaaggagct gacacaggac 2580

gatctgcggt acattgacaa cttcagcatc ttcaacgaga agaataagac catcgacatc 2640

attaaggata agaggttcac agtcgataag ttccagttcc acgtgcctat caccatgaat 2700

ttcaaggcca caggcggctc atacattaac cagaccgtcc tggagtacct ccagaacaat 2760

ccggaggtga agatcattgg cctcgaccgg ggcgagaggc acctggtgta cctcaccctg 2820

attgatcagc agggcaatat cctcaagcag gagtccctga acaccattac agactccaag 2880

atcagcacac cataccataa gctcctggac aacaaggaga atgagcgcga tctcgcgcgg 2940

aagaactggg gcaccgtcga gaatatcaag gagctgaagg agggctacat ttcccaggtg 3000

gtccataaga tcgccacact catgctggag gagaatgcga ttgtggtcat ggaggatctc 3060

aacttcggct tcaagagggg ccgcttcaag gtggagaagc agatctacca gaagctcgag 3120

aagatgctga ttgacaagct caattacctc gtcctgaagg ataagcagcc tcaggagctg 3180

ggcggcctct acaacgccct ccagctgacc aataagttcg agtcattcca gaagatgggc 3240

aagcagtctg gcttcctctt ctacgtgccg gcgtggaaca ccagcaagat tgacccaacc 3300

acaggcttcg tcaattactt ctacacaaag tacgagaacg tggataaggc caaggcgttc 3360

ttcgagaagt tcgaggccat ccgcttcaat gcggagaaga agtacttcga gttcgaggtc 3420

aagaagtact cagacttcaa tccaaaggcg gagggaaccc agcaggcgtg gaccatttgc 3480

acatacggcg agaggatcga gacaaagcgc cagaaggatc agaacaataa gttcgtgtct 3540

accccaatta atctcacaga gaagatcgag gacttcctgg gcaagaatca gatcgtctac 3600

ggcgatggca actgcatcaa gtcccagatt gccagcaagg acgataaggc gttcttcgag 3660

acactcctgt actggttcaa gatgaccctc cagatgcgga actcagagac aaggacagac 3720

atcgattacc tgatttctcc agtgatgaac gacaatggca ccttctacaa ttccagggat 3780

tacgagaagc tcgagaaccc gacactgcca aaggacgccg atgcgaatgg cgcctaccat 3840

atcgcgaaga agggcctcat gctcctgaac aagattgacc aggccgatct caccaagaag 3900

gtcgacctgt ccatcagcaa tcgcgattgg ctgcagttcg tgcagaagaa caag 3954

<210> 3
<211> 4
<212> PRT
<213> Artificial Sequence

<220>

<223>  linker

<400>  3

Ser Gly Gly Ser
1


<210>  4
<211>  7
<212>  PRT
<213>  Artificial Sequence

<220>
<223>  SV40 nuclear localization sequence

<400>  4

Pro Lys Lys Lys Arg Lys Val
1               5


<210>  5
<211>  16
<212>  PRT
<213>  Artificial Sequence

<220>
<223>  nucleoplasmin nuclear localization sequence

<400>  5

Lys Arg Pro Ala Ala Thr Lys Lys Ala Gly Gln Ala Lys Lys Lys Lys
1               5                   10                  15


<210>  6
<211>  1349
<212>  PRT
<213>  Artificial Sequence

<220>
<223>  FbCas12a amino acid sequence with nuclear localization sequence

<400>  6

Met Thr Asn Lys Phe Thr Asn Gln Tyr Ser Leu Ser Lys Thr Leu Arg
1               5                   10                  15


Phe Glu Leu Ile Pro Gln Gly Lys Thr Leu Glu Phe Ile Gln Glu Lys
            20                  25                  30


Gly Leu Leu Ser Gln Asp Lys Gln Arg Ala Glu Ser Tyr Gln Glu Met
        35                  40                  45


Lys Lys Thr Ile Asp Lys Phe His Lys Tyr Phe Ile Asp Leu Ala Leu
    50                  55                  60


Ser Asn Ala Lys Leu Thr His Leu Glu Thr Tyr Leu Glu Leu Tyr Asn

65                  70                  75                  80

Lys Ser Ala Glu Thr Lys Lys Glu Gln Lys Phe Lys Asp Asp Leu Lys
                85                  90                  95

Lys Val Gln Asp Asn Leu Arg Lys Glu Ile Val Lys Ser Phe Ser Asp
            100                 105                 110

Gly Asp Ala Lys Ser Ile Phe Ala Ile Leu Asp Lys Lys Glu Leu Ile
        115                 120                 125

Thr Val Glu Leu Glu Lys Trp Phe Glu Asn Asn Glu Gln Lys Asp Ile
    130                 135                 140

Tyr Phe Asp Glu Lys Phe Lys Thr Phe Thr Thr Tyr Phe Thr Gly Phe
145                 150                 155                 160

His Gln Asn Arg Lys Asn Met Tyr Ser Val Glu Pro Asn Ser Thr Ala
                165                 170                 175

Ile Ala Tyr Arg Leu Ile His Glu Asn Leu Pro Lys Phe Leu Glu Asn
            180                 185                 190

Ala Lys Ala Phe Glu Lys Ile Lys Gln Val Glu Ser Leu Gln Val Asn
        195                 200                 205

Phe Arg Glu Leu Met Gly Glu Phe Gly Asp Glu Gly Leu Ile Phe Val
    210                 215                 220

Asn Glu Leu Glu Glu Met Phe Gln Ile Asn Tyr Tyr Asn Asp Val Leu
225                 230                 235                 240

Ser Gln Asn Gly Ile Thr Ile Tyr Asn Ser Ile Ile Ser Gly Phe Thr
                245                 250                 255

Lys Asn Asp Ile Lys Tyr Lys Gly Leu Asn Glu Tyr Ile Asn Asn Tyr
            260                 265                 270

Asn Gln Thr Lys Asp Lys Lys Asp Arg Leu Pro Lys Leu Lys Gln Leu
        275                 280                 285

Tyr Lys Gln Ile Leu Ser Asp Arg Ile Ser Leu Ser Phe Leu Pro Asp
    290                 295                 300

Ala Phe Thr Asp Gly Lys Gln Val Leu Lys Ala Ile Phe Asp Phe Tyr
305                 310                 315                 320

Lys Ile Asn Leu Leu Ser Tyr Thr Ile Glu Gly Gln Glu Glu Ser Gln
                325                 330                 335

Asn Leu Leu Leu Leu Ile Arg Gln Thr Ile Glu Asn Leu Ser Ser Phe
            340                 345                 350

Asp Thr Gln Lys Ile Tyr Leu Lys Asn Asp Thr His Leu Thr Thr Ile
        355                 360                 365

Ser Gln Gln Val Phe Gly Asp Phe Ser Val Phe Ser Thr Ala Leu Asn
    370                 375                 380

Tyr Trp Tyr Glu Thr Lys Val Asn Pro Lys Phe Glu Thr Glu Tyr Ser
385                 390                 395                 400

Lys Ala Asn Glu Lys Lys Arg Glu Ile Leu Asp Lys Ala Lys Ala Val
                405                 410                 415

Phe Thr Lys Gln Asp Tyr Phe Ser Ile Ala Phe Leu Gln Glu Val Leu
            420                 425                 430

Ser Glu Tyr Ile Leu Thr Leu Asp His Thr Ser Asp Ile Val Lys Lys
        435                 440                 445

His Ser Ser Asn Cys Ile Ala Asp Tyr Phe Lys Asn His Phe Val Ala
    450                 455                 460

Lys Lys Glu Asn Glu Thr Asp Lys Thr Phe Asp Phe Ile Ala Asn Ile
465                 470                 475                 480

Thr Ala Lys Tyr Gln Cys Ile Gln Gly Ile Leu Glu Asn Ala Asp Gln
                485                 490                 495

Tyr Glu Asp Glu Leu Lys Gln Asp Gln Lys Leu Ile Asp Asn Leu Lys
            500                 505                 510

Phe Phe Leu Asp Ala Ile Leu Glu Leu Leu His Phe Ile Lys Pro Leu
        515                 520                 525

His Leu Lys Ser Glu Ser Ile Thr Glu Lys Asp Thr Ala Phe Tyr Asp
    530                 535                 540

Val Phe Glu Asn Tyr Tyr Glu Ala Leu Ser Leu Leu Thr Pro Leu Tyr
545                 550                 555                 560

Asn Met Val Arg Asn Tyr Val Thr Gln Lys Pro Tyr Ser Thr Glu Lys
                565                 570                 575

Ile Lys Leu Asn Phe Glu Asn Ala Gln Leu Leu Asn Gly Trp Asp Ala
            580                 585                 590

Asn Lys Glu Gly Asp Tyr Leu Thr Thr Ile Leu Lys Lys Asp Gly Asn
        595                 600                 605

Tyr Phe Leu Ala Ile Met Asp Lys Lys His Asn Lys Ala Phe Gln Lys
    610                 615                 620

Phe Pro Glu Gly Lys Glu Asn Tyr Glu Lys Met Val Tyr Lys Leu Leu
625                 630                 635                 640

Pro Gly Val Asn Lys Met Leu Pro Lys Val Phe Phe Ser Asn Lys Asn
                645                 650                 655

Ile Ala Tyr Phe Asn Pro Ser Lys Glu Leu Leu Glu Asn Tyr Lys Lys
            660                 665                 670

Glu Thr His Lys Lys Gly Asp Thr Phe Asn Leu Glu His Cys His Thr
        675                 680                 685

Leu Ile Asp Phe Phe Lys Asp Ser Leu Asn Lys His Glu Asp Trp Lys
    690                 695                 700

Tyr Phe Asp Phe Gln Phe Ser Glu Thr Lys Ser Tyr Gln Asp Leu Ser
705                 710                 715                 720

Gly Phe Tyr Arg Glu Val Glu His Gln Gly Tyr Lys Ile Asn Phe Lys
                725                 730                 735

Asn Ile Asp Ser Glu Tyr Ile Asp Gly Leu Val Asn Glu Gly Lys Leu
            740                 745                 750

Phe Leu Phe Gln Ile Tyr Ser Lys Asp Phe Ser Pro Phe Ser Lys Gly
        755                 760                 765

Lys Pro Asn Met His Thr Leu Tyr Trp Lys Ala Leu Phe Glu Glu Gln
    770                 775                 780

Asn Leu Gln Asn Val Ile Tyr Lys Leu Asn Gly Gln Ala Glu Ile Phe
785                 790                 795                 800

Phe Arg Lys Ala Ser Ile Lys Pro Lys Asn Ile Ile Leu His Lys Lys
                805                 810                 815

Lys Ile Lys Ile Ala Lys Lys His Phe Ile Asp Lys Lys Thr Lys Thr
            820                 825                 830

Ser Glu Ile Val Pro Val Gln Thr Ile Lys Asn Leu Asn Met Tyr Tyr
        835                 840                 845

Gln Gly Lys Ile Ser Glu Lys Glu Leu Thr Gln Asp Asp Leu Arg Tyr
    850                 855                 860

Ile Asp Asn Phe Ser Ile Phe Asn Glu Lys Asn Lys Thr Ile Asp Ile
865                 870                 875                 880

Ile Lys Asp Lys Arg Phe Thr Val Asp Lys Phe Gln Phe His Val Pro
                885                 890                 895

Ile Thr Met Asn Phe Lys Ala Thr Gly Gly Ser Tyr Ile Asn Gln Thr
            900                 905                 910

Val Leu Glu Tyr Leu Gln Asn Asn Pro Glu Val Lys Ile Ile Gly Leu
        915                 920                 925

Asp Arg Gly Glu Arg His Leu Val Tyr Leu Thr Leu Ile Asp Gln Gln
    930                 935                 940

Gly Asn Ile Leu Lys Gln Glu Ser Leu Asn Thr Ile Thr Asp Ser Lys
945                 950                 955                 960

Ile Ser Thr Pro Tyr His Lys Leu Leu Asp Asn Lys Glu Asn Glu Arg
                965                 970                 975

Asp Leu Ala Arg Lys Asn Trp Gly Thr Val Glu Asn Ile Lys Glu Leu
            980                 985                 990

Lys Glu Gly Tyr Ile Ser Gln Val Val His Lys Ile Ala Thr Leu Met
        995                 1000                1005

Leu Glu Glu Asn Ala Ile Val Val Met Glu Asp Leu Asn Phe Gly
    1010                1015                1020

Phe Lys Arg Gly Arg Phe Lys Val Glu Lys Gln Ile Tyr Gln Lys
    1025                1030                1035

Leu Glu Lys Met Leu Ile Asp Lys Leu Asn Tyr Leu Val Leu Lys
    1040                1045                1050

Asp Lys Gln Pro Gln Glu Leu Gly Gly Leu Tyr Asn Ala Leu Gln
    1055                1060                1065

Leu Thr Asn Lys Phe Glu Ser Phe Gln Lys Met Gly Lys Gln Ser

1070                1075                1080

Gly Phe Leu Phe Tyr Val Pro Ala Trp Asn Thr Ser Lys Ile Asp
    1085                1090                1095

Pro Thr Thr Gly Phe Val Asn Tyr Phe Tyr Thr Lys Tyr Glu Asn
    1100                1105                1110

Val Asp Lys Ala Lys Ala Phe Phe Glu Lys Phe Glu Ala Ile Arg
    1115                1120                1125

Phe Asn Ala Glu Lys Lys Tyr Phe Glu Phe Glu Val Lys Lys Tyr
    1130                1135                1140

Ser Asp Phe Asn Pro Lys Ala Glu Gly Thr Gln Gln Ala Trp Thr
    1145                1150                1155

Ile Cys Thr Tyr Gly Glu Arg Ile Glu Thr Lys Arg Gln Lys Asp
    1160                1165                1170

Gln Asn Asn Lys Phe Val Ser Thr Pro Ile Asn Leu Thr Glu Lys
    1175                1180                1185

Ile Glu Asp Phe Leu Gly Lys Asn Gln Ile Val Tyr Gly Asp Gly
    1190                1195                1200

Asn Cys Ile Lys Ser Gln Ile Ala Ser Lys Asp Asp Lys Ala Phe
    1205                1210                1215

Phe Glu Thr Leu Leu Tyr Trp Phe Lys Met Thr Leu Gln Met Arg
    1220                1225                1230

Asn Ser Glu Thr Arg Thr Asp Ile Asp Tyr Leu Ile Ser Pro Val
    1235                1240                1245

Met Asn Asp Asn Gly Thr Phe Tyr Asn Ser Arg Asp Tyr Glu Lys
    1250                1255                1260

Leu Glu Asn Pro Thr Leu Pro Lys Asp Ala Asp Ala Asn Gly Ala
    1265                1270                1275

Tyr His Ile Ala Lys Lys Gly Leu Met Leu Leu Asn Lys Ile Asp
    1280                1285                1290

Gln Ala Asp Leu Thr Lys Lys Val Asp Leu Ser Ile Ser Asn Arg
    1295                1300                1305

Asp Trp Leu Gln Phe Val Gln Lys Asn Lys Ser Gly Gly Ser Pro
    1310                1315                1320

Lys Lys Lys Arg Lys Val Ser Gly Gly Ser Lys Arg Pro Ala Ala
    1325                1330                1335

Thr Lys Lys Ala Gly Gln Ala Lys Lys Lys Lys
    1340                1345

<210> 7
<211> 4047
<212> DNA
<213> Artificial Sequence

<220>
<223> coding sequence of FbCas12a with nuclear localization sequence

<400> 7
atgaccaaca agttcacaaa tcagtactcc ctgagcaaga ccctccgctt cgagctgatt 60

ccgcagggca agacactcga gttcatccag gagaagggcc tcctgtccca ggacaagcag 120

agggccgaga gctaccagga gatgaagaag accattgaca agttccacaa gtacttcatc 180

gatctcgccc tgtcaaatgc gaagctcacc catctggaga catacctcga gctgtacaac 240

aagtctgcgg agacaaagaa ggagcagaag ttcaaggacg atctcaagaa ggtccaggac 300

aacctgagga aggagatcgt gaagtcattc tctgacggcg atgccaagag catcttcgcg 360

attctggata agaaggagct gatcaccgtg gagctggaga agtggttcga gaacaatgag 420

cagaaggaca tctacttcga tgagaagttc aagaccttca ccacatactt cacaggcttc 480

caccagaaca ggaagaatat gtactcagtc gagccaaact ctacagccat cgcgtaccgc 540

ctcattcatg agaacctccc taagttcctg gagaatgcca aggcgttcga gaagatcaag 600

caggtggagt ccctccaggt caatttccgc gagctgatgg gcgagttcgg cgacgagggc 660

ctcatcttcg tcaacgagct ggaggagatg ttccagatta actactacaa tgatgtgctc 720

agccagaacg gcatcaccat ctacaattca atcatttctg gcttcacaaa gaacgacatt 780

aagtacaagg gcctgaacga gtacatcaac aattacaatc agaccaagga caagaaggat 840

cgcctcccga agctgaagca gctctacaag cagatcctct ccgaccggat ttccctgagc 900

ttcctcccag acgccttcac cgatggcaag caggtgctga aggcgatttt cgatttctac 960

aagatcaatc tcctgtccta cacaatcgag ggccaggagg agagccagaa tctcctgctc 1020

ctgatccggc agacaattga gaacctctcc agcttcgaca cccagaagat ctacctcaag 1080

aacgatacac acctgaccac aatttcccag caggtgttcg gcgatttctc cgtcttcagc 1140

accgccctca actactggta cgagacaaaa gtgaatccga agttcgagac agagtacagc 1200

aaggcgaacg agaagaagag ggagatcctg gacaaggcca aggcggtctt caccaagcag 1260

gattacttct caattgcctt cctccaggag gtgctgtctg agtacattct caccctggac 1320

cacacatccg atatcgtcaa gaagcattca tctaactgca ttgccgacta cttcaagaat 1380

catttcgtcg cgaagaagga gaacgagaca gacaagacat tcgatttcat cgccaatatt 1440

acagcgaagt accagtgcat ccagggcatt ctcgagaacg ccgaccagta cgaggatgag 1500

ctgaagcagg accagaagct cattgataat ctgaagttct tcctcgatgc gatcctcgag 1560

ctgctgcact tcatcaagcc actccatctg aagtcagagt ctatcaccga gaaggacaca 1620

gccttctacg atgtgttcga gaactactac gaggcgctct ccctcctgac ccctctgtac 1680

aacatggtga ggaattacgt cacccagaag ccgtacagca cagagaagat caagctcaac 1740

ttcgagaatg cccagctcct gaacggctgg gacgcgaata aggagggcga ttacctcacc 1800

acaatcctga agaaggacgg caattacttc ctcgccatta tggataagaa gcacaacaag 1860

gcgttccaga agttccctga gggcaaggag aattacgaga agatggtgta caagctcctg 1920

ccaggcgtca acaagatgct ccctaaggtg ttcttctcca acaagaatat cgcctacttc 1980

aatccgagca aggagctgct cgagaactac aagaaggaga cacacaagaa gggcgacaca 2040

ttcaatctcg agcactgcca taccctgatc gacttcttca aggattccct caacaagcat 2100

gaggactgga agtacttcga tttccagttc tcagagacaa agtcttacca ggatctgagc 2160

ggcttctacc gcgaggtgga gcaccagggc tacaagatca acttcaagaa tattgactca 2220

gagtacatcg atggcctcgt caatgagggc aagctcttcc tgttccagat ctactctaag 2280

gacttctcac ctttctctaa gggcaagccg aacatgcata ccctctactg gaaggccctg 2340

ttcgaggagc agaacctcca gaatgtgatc tacaagctga atggccaggc cgagattttc 2400

ttccggaagg cgtcaatcaa gccaaagaac atcattctcc acaagaagaa gatcaagatt 2460

gcgaagaagc atttcatcga taagaagacc aagacatctg agattgtgcc tgtccagacc 2520

atcaagaacc tcaatatgta ctaccagggc aagatttccg agaaggagct gacacaggac 2580

gatctgcggt acattgacaa cttcagcatc ttcaacgaga agaataagac catcgacatc 2640

attaaggata agaggttcac agtcgataag ttccagttcc acgtgcctat caccatgaat 2700

ttcaaggcca caggcggctc atacattaac cagaccgtcc tggagtacct ccagaacaat 2760

ccggaggtga agatcattgg cctcgaccgg ggcgagaggc acctggtgta cctcaccctg 2820

attgatcagc agggcaatat cctcaagcag gagtccctga acaccattac agactccaag 2880

atcagcacac cataccataa gctcctggac aacaaggaga atgagcgcga tctcgcgcgg 2940

aagaactggg gcaccgtcga gaatatcaag gagctgaagg agggctacat ttcccaggtg 3000

gtccataaga tcgccacact catgctggag gagaatgcga ttgtggtcat ggaggatctc 3060

aacttcggct tcaagagggg ccgcttcaag gtggagaagc agatctacca gaagctcgag 3120

aagatgctga ttgacaagct caattacctc gtcctgaagg ataagcagcc tcaggagctg 3180

ggcggcctct acaacgccct ccagctgacc aataagttcg agtcattcca gaagatgggc 3240

aagcagtctg gcttcctctt ctacgtgccg gcgtggaaca ccagcaagat tgacccaacc 3300

acaggcttcg tcaattactt ctacacaaag tacgagaacg tggataaggc caaggcgttc 3360

ttcgagaagt tcgaggccat ccgcttcaat gcggagaaga agtacttcga gttcgaggtc 3420

aagaagtact cagacttcaa tccaaaggcg gagggaaccc agcaggcgtg gaccatttgc 3480

acatacggcg agaggatcga gacaaagcgc cagaaggatc agaacaataa gttcgtgtct 3540

accccaatta atctcacaga gaagatcgag gacttcctgg gcaagaatca gatcgtctac 3600

ggcgatggca actgcatcaa gtcccagatt gccagcaagg acgataaggc gttcttcgag 3660

acactcctgt actggttcaa gatgaccctc cagatgcgga actcagagac aaggacagac 3720

atcgattacc tgatttctcc agtgatgaac gacaatggca ccttctacaa ttccagggat 3780

tacgagaagc tcgagaaccc gacactgcca aaggacgccg atgcgaatgg cgcctaccat 3840

atcgcgaaga agggcctcat gctcctgaac aagattgacc aggccgatct caccaagaag 3900

gtcgacctgt ccatcagcaa tcgcgattgg ctgcagttcg tgcagaagaa caagtccggc 3960

ggcagcccaa agaagaagag gaaggtgagc ggcggcagca agcggccagc ggcgacgaag 4020

aaggcggggc aggcgaagaa gaagaag 4047

<210> 8
<211> 3687
<212> DNA
<213> Artificial Sequence

<220>
<223> codon-optimized LbCas12a coding sequence

<400> 8
atgtcaaagc tcgagaaatt caccaactgt tattcgttga gcaaaacact gcggtttaaa 60

gcgattccag tcggcaagac tcaagagaat atagacaata agcggctgtt ggtggaagat 120

gaaaagcgcg cggaagacta caaaggggtg aagaagttgt tggacagata ctacctctct 180

tttatcaatg atgtcttgca ctcaatcaaa ttgaagaatc tgaacaacta catctccctc 240

ttcagaaaga aaacaaggac agaaaaggag aataaggaac ttgaaaattt ggagatcaat 300

ctgaggaaag agatcgcgaa agcctttaaa ggcaacgaag gatacaaaag tctgttcaag 360

aaggatataa ttgagacaat tttgccagag ttcctcgatg acaaggacga gattgcgctg 420

gtcaattcgt tcaacggatt cacaacagca ttcacaggct tctttgataa tcgggaaaat 480

atgttctctg aggaggcaaa gtccacttct attgcgttca ggtgtatcaa tgagaatctc 540

actaggtaca tttccaacat ggatatcttt gagaaggttg acgcaatttt tgacaagcac 600

gaagttcagg agattaagga gaagatcctc aattccgatt atgacgttga ggacttcttc 660

gaaggtgagt tttttaattt cgtgctcact caagagggta tcgacgtgta taatgcgatc 720

atcggtgggt tcgtgactga gtccggtgaa aagattaagg gattgaacga gtatatcaac 780

ctttacaacc aaaagacgaa acagaagctg ccaaagttca agcctcttta caaacaggtt 840

ctttcagacc gcgagtcact ctcgttctat ggggagggct acacttcgga tgaggaagtc 900

ctggaggtgt tcaggaatac tctcaataag aattcggaga ttttctcttc tataaaaaaa 960

ctggaaaagt tgtttaagaa ttttgacgaa tactctagcg ccggcatatt tgtgaaaaac 1020

ggcccggcca tatcaacgat aagtaaagat atcttcggcg aatggaacgt gatcagagac 1080

aaatggaacg cggagtatga cgatattcac ctgaagaaga aggctgtcgt aacggagaag 1140

tacgaggatg atcgcaggaa aagcttcaaa aagatcggaa gtttcagcct ggaacagttg 1200

caggagtatg ctgacgccga tcttagcgtc gtcgagaagt tgaaggagat aatcatccaa 1260

aaggtcgacg agatatataa agtctatgga tcaagtgaaa aactgttcga cgccgacttc 1320

gttttggaga agtccctgaa gaagaacgac gctgttgttg ccattatgaa ggatctgctc 1380

gacagcgtga agagtttcga gaactatatt aaggcttttt tcggggaggg gaaggagact 1440

aacagagatg agtccttcta cggagacttc gtcctcgcgt acgatatact ccttaaggta 1500

gaccacatct acgacgcaat cagaaattac gtgacacaaa agccgtacag caaggacaag 1560

ttcaaactct acttccagaa cccccagttc atgggcggct gggacaagga caaggaaacg 1620

gattacaggg ctacgatcct gaggtatggt tcaaaatact acttggcgat tatggacaag 1680

aagtacgcca agtgtctcca gaagattgac aaagacgatg tcaatggcaa ttatgagaag 1740

atcaactaca agctgcttcc gggtccgaac aagatgctcc caaaggtttt cttcagcaag 1800

aaatggatgg cctactataa cccaagcgag gacatccaga agatttataa gaacggtacg 1860

ttcaagaagg gcgacatgtt caatcttaac gactgtcaca agctgatcga cttcttcaaa 1920

gactcaatta gccggtaccc aaagtggtct aacgcctatg acttcaactt ttcggaaacc 1980

gagaagtaca aggatatagc cggattttat agagaggtgg aagagcaggg ctacaaggtg 2040

tcattcgagt ccgccagcaa gaaggaagtg gacaagctcg tggaagaggg taagctctac 2100

atgttccaga tttataataa agactttagc gataagagcc acgggacacc taatctccac 2160

acaatgtatt tcaagctgct cttcgacgag aataaccacg gccaaatcag gttgtcagga 2220

ggggctgaac tcttcatgcg gcgcgctagc cttaagaagg aggagcttgt agtccaccct 2280

gcgaatagtc caattgcgaa taagaacccg gacaatccta aaaagactac aacattgagc 2340

tacgacgtgt acaaggataa gaggttttcc gaggatcagt acgagctcca catcccgatt 2400

gcgatcaaca agtgcccaaa gaatattttc aagataaaca cagaggtgcg tgtactcctg 2460

aagcatgacg acaatcctta cgtcattggg attgatcggg gcgagaggaa cctcctctat 2520

attgtggtgg tggacgggaa ggggaacata gtcgaacagt actcccttaa cgaaataatt 2580

aacaatttca acggcatccg tatcaagacc gactaccatt cgttgctgga caagaaggag 2640

aaggagagat ttgaggcgcg gcaaaattgg acaagtatcg agaacatcaa ggaactcaaa 2700

gcaggttata tctctcaagt tgtgcataag atatgcgagc tggttgagaa gtatgacgca 2760

gtgatcgctc ttgaggacct caactcgggc tttaagaatt ctagagttaa agtggagaag 2820

caggtctatc aaaagttcga gaagatgctt atagataagc tcaactacat ggtcgataag 2880

aaatcgaacc catgtgccac cggcggcgca ctcaaaggtt accaaataac aaacaaattc 2940

gagtccttca aatcgatgag tactcagaat gggttcatat tttatatacc ggcgtggctt 3000

acgtctaaga tcgacccgtc aactggtttt gtcaacctgt tgaagacgaa atacacgtcc 3060

attgccgatt cgaaaaagtt catatctagt tttgatcgta ttatgtacgt cccagaggaa 3120

gatcttttcg agtttgctct cgactacaaa aacttttcgc ggaccgatgc ggattacatt 3180

aaaaaatgga aactctattc gtacggcaac agaatcagga tttttcgcaa ccctaagaag 3240

aataacgtct ttgattggga ggaagtttgc ttgactagcg cgtacaagga gctctttaat 3300

aagtatggca ttaactacca acagggtgat atcagagcac tgctttgcga acaatctgac 3360

aaggctttct actcatcctt catggctttg atgagcctga tgctccagat gagaaattca 3420

attacaggca gaaccgacgt ggatttcttg atctccccgg ttaaaaattc tgatggcatc 3480

ttttacgata gcaggaacta tgaagcgcaa gagaatgcga ttctgccaaa aaatgcagac 3540

gccaacggtg cctataacat cgccaggaaa gtcctgtggg cgatcggcca gttcaaaaag 3600

gccgaagacg aaaaattgga caaggtcaaa atcgctatca gcaacaaaga gtggctggag 3660

tatgctcaga catccgtaaa gcattag 3687

<210> 9
<211> 36
<212> DNA
<213> Artificial Sequence

<220>
<223> DR sequence of FbCas12a

<400> 9
gtttaaaacc actttaaaat ttctactatt gtagat 36

<210> 10
<211> 19
<212> RNA
<213> Artificial Sequence

<220>
<223> predicted crRNA scaffold sequence of FbCas12a

<400> 10
aauuucuacu auuguagau 19

<210>  11
<211>  21
<212>  RNA
<213>  Artificial Sequence

<220>
<223>  crRNA scaffold sequence of LbCas12a

<400>  11
uaauuucuac uaaguguaga u                                                21


<210>  12
<211>  19
<212>  DNA
<213>  Artificial Sequence

<220>
<223>  coding sequence of predicted crRNA scaffold sequence of FbCas12a

<400>  12
aatttctact attgtagat                                                   19


<210>  13
<211>  21
<212>  DNA
<213>  Artificial Sequence

<220>
<223>  coding sequence of crRNA scaffold sequence of LbCas12a

<400>  13
taatttctac taagtgtaga t                                                21


<210>  14
<211>  162
<212>  DNA
<213>  Artificial Sequence

<220>
<223>  expression cassette of FbCas12a crRNA in combination with ribozyme

<400>  14
aagcttaaat tactgatgag tccgtgagga cgaaacgagt aagctcgtca atttctacta      60

ttgtagattg agaccctgca gggtctctgg ccggcatggt cccagcctcc tcgctggcgc     120

cggctgggca acatgcttcg gcatggcgaa tgggaccccg gg                        162


<210>  15
<211>  164
<212>  DNA
<213>  Artificial Sequence

<220>
<223>  expression cassette of LbCas12a crRNA in combination with ribozyme

<400>  15
aagcttaaat tactgatgag tccgtgagga cgaaacgagt aagctcgtct aatttctact      60

aagtgtagat tgagaccctg cagggtctct ggccggcatg gtcccagcct cctcgctggc 120

gccggctggg caacatgctt cggcatggcg aatgggaccc cggg 164


<210> 16
<211> 27
<212> DNA
<213> Artificial Sequence

<220>
<223> target-EPSPS05

<400> 16
tttggtacta aatatacaat cccttgg 27


<210> 17
<211> 24
<212> DNA
<213> Artificial Sequence

<220>
<223> target-OsCDC48

<400> 17
tttattcaga ttacatatgg ttag 24


<210> 18
<211> 27
<212> DNA
<213> Artificial Sequence

<220>
<223> target-OsDEP1T3

<400> 18
tttcaaatgg atctaaacag ggcctta 27


<210> 19
<211> 24
<212> DNA
<213> Artificial Sequence

<220>
<223> target-OsPDS

<400> 19
tttggagtga aatctcttgt ctta 24


<210> 20
<211> 27
<212> DNA
<213> Artificial Sequence

<220>
<223> target-OsEpspsC02

<400> 20

tttatgaaaa tatgtatgga attcatg                                          27

<210>  21
<211>  27
<212>  DNA
<213>  Artificial Sequence

<220>
<223>  SP1-F

<400>  21
agatgtacta aatatacaat cccttgg                                          27

<210>  22
<211>  27
<212>  DNA
<213>  Artificial Sequence

<220>
<223>  SP1-R

<400>  22
aaacccaagg gattgtatat ttagtac                                          27

<210>  23
<211>  24
<212>  DNA
<213>  Artificial Sequence

<220>
<223>  SP2-F

<400>  23
agatttcaga ttacatatgg ttag                                             24

<210>  24
<211>  24
<212>  DNA
<213>  Artificial Sequence

<220>
<223>  SP2-R

<400>  24
aaacctaacc atatgtaatc tgaa                                             24

<210>  25
<211>  27
<212>  DNA
<213>  Artificial Sequence

<220>
<223>  SP3-F

<400>  25
agataaatgg atctaaacag ggcctta                                          27

<210>  26
<211>  27
<212>  DNA
<213>  Artificial Sequence

<220>
<223>  SP3-R

<400>  26
aaacattggc cctgtttaga tccattt                                          27


<210>  27
<211>  24
<212>  DNA
<213>  Artificial Sequence

<220>
<223>  SP4-F

<400>  27
agatgagtga aatctcttgt ctta                                             24


<210>  28
<211>  24
<212>  DNA
<213>  Artificial Sequence

<220>
<223>  SP4-R

<400>  28
aaactaagac aagagatttc actc                                             24


<210>  29
<211>  27
<212>  DNA
<213>  Artificial Sequence

<220>
<223>  SP5-F

<400>  29
agattgaaaa tatgtatgga attcatg                                          27


<210>  30
<211>  27
<212>  DNA
<213>  Artificial Sequence

<220>
<223>  SP5-R

<400>  30
aaaccatgaa ttccatacat attttca                                          27


<210>  31
<211>  43
<212>  DNA

<213>  Artificial Sequence

<220>
<223>  5′ ribozyme sequence



<220>
<221>  misc_feature
<222>  (1)..(6)
<223>  n is a, c, g, or t

<400>  31
nnnnnnctga tgagtccgtg aggacgaaac gagtaagctc gtc                          43



<210>  32
<211>  68
<212>  DNA
<213>  Artificial Sequence

<220>
<223>  3′ ribozyme sequence

<400>  32
ggccggcatg gtcccagcct cctcgctggc gccggctggg caacatgctt cggcatggcg      60

aatgggac                                                                 68